# EUROPEAN PATENT APPLICATION

(11) **EP 1 048 343 A2**
(43) Date of publication of application: **02.11.2000**
(21) Application number: 00106188.6
(22) Date of filing: 22.03.2000
(51) Int. Cl.: B01J 8/00, B01J 8/06, C01B 3/38, C01C 1/04, C07C 29/152

(54) **Heat exchanger type reactor**

(30) Priority: 26.04.1999 JP 11793799; 02.03.2000 JP 2000057257
(71) Applicant: TOYO ENGINEERING CORPORATION, Chiyoda-ku, Tokyo 100 (JP)
(72) Inventor: Sakai, Kenji, Sakura-shi, Chiba (JP)
(74) Representative: Luderschmidt, Schüler & Partner GbR

(57) **Abstract**

A heat-exchanger type reactor which has a plurality of tubes holding a catalyst, a shell section through which a heat-transfer medium is passed to carry out heat-transfer with a reaction fluid in said tubes, and upper and lower tube sheets, the upper ends of said tubes being joined to said upper tube sheet by way of first expansion joints fixed to the upper side of said upper tube sheet, the lower ends of said tubes being fixed directly to the floatable lower tube sheet, a floatable room being formed which is partitioned by said lower tube sheet and an inner end plate (inner head) joined to the lower side thereof and has an opening in the lower part, and said opening being joined by way of a second expansion joint to a tube-side outlet to the outside of the reactor.

## Description

### Field of the Invention

The invention relates to an improved heat-exchanger type reactor, and specifically to an improved heat-exchanger type reactor for carrying out an endothermic or exothermic reaction.

### Description of the Related Art

Heat-exchanger type reactors are widely used to carry out a variety of endothermic reactions, such as steam reforming reactions for hydrocarbons, or a variety of exothermic reactions, such as ammonia synthesis and methanol synthesis reactions. Conditions required for such reactors include (1) a structure that can hold a catalyst inside the tubes, (2) a structure that can absorb the differential thermal expansion between the tubes and the shell in the reactor due to the temperature difference between them, and (3) a structure that can cope with the differential thermal expansion between tubes, which is caused by the temperature difference between tubes, produced by the difference in reaction and heat-transfer conditions between tubes, the difference being due to the tolerance in tube inner diameter in the reactor, the difference in catalyst packing density in each tube, the difference in catalyst activity, the uneven distribution of a reaction gas flowing through the tubes, the uneven distribution of a heat-transfer medium flowing through the shell-section, and the like.

Conventional heat exchanger type reactors cannot meet all of these requirements regardless of their forms.

For example, for heat-exchanger type reactors of the fixed tube-sheet type, in which the tube sheets are fixed to the wall of the reactor, it is easy to have a structure that can hold a catalyst inside the tubes, but they cannot cope with the differential thermal expansion between the shell and the tubes or between tubes.

For heat-exchanger type reactors of the U-tube type, in which the tubes are bent in the form of U-tubes, it is easy to cope with the aforementioned differential thermal expansions, but is difficult to have a structure that can pack and draw out a catalyst inside the tubes easily.

Although it is easy for heat-exchanger type reactors of the floating head type, in which a tube sheet is movable, to have a structure that can hold a catalyst inside the tubes and to cope with the differential thermal expansion between the shell and the tubes, it is not possible for them to cope with the differential thermal expansion between tubes.

There is a heat-exchanger type reactor having tubes bundled in a vessel, in which manifolds (headers) are provided at the upper and lower parts of the reactor, and each tube is tied to the manifolds by way of bent-tubes so as to absorb the differential thermal expansion of the tubes. However, it is difficult for the reactor to have a structure that can pack or draw out a catalyst easily.

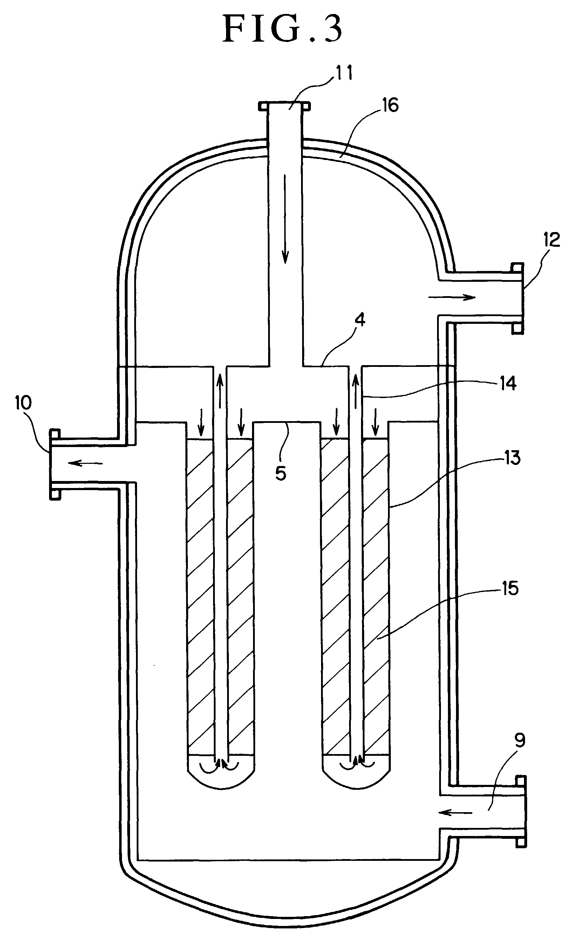

The reactor shown in Fig. 3 can meet all of the foregoing requirements by employing sheath tubes (bayonet tubes). In Fig. 3, interpolated tubes 14 are joined to an upper tube sheet 4 provided inside the reactor 1. To a lower tube sheet 5 are fitted bayonet tubes 13 with the lower ends closed so as to surround the interpolated tubes 14. A catalyst is packed in the spaces between the outer walls of the interpolated tubes 14 and the inner walls of the bayonet tubes. A heat-transfer medium is introduced through a shell-side inlet 9 into the shell-section, that is the space formed by the outer walls of the lower tube sheet 5 and the bayonet tubes 13 and the inner wall of the reactor 1, and discharged through a shell-side outlet 10, to transfer heat to a reaction gas through the bayonet tubes 13. The reaction gas is fed through a tube-side inlet 11, sent through the catalyst layers in the bayonet tubes 13, and discharged through a tube-side outlet 12 via the interpolated tubes 14.

As described above, in the heat-exchanger type reactor shown in Fig. 3, neither the bottoms of the bayonet tubes 13 nor the lower ends of the interpolated tubes 14 are fixed, so that it is possible to absorb the differential thermal expansion between tubes and that between the shell and the tubes. However, the reactor have many defects in that it is not easy to pack or draw out a catalyst, that a reaction gas before and after the reaction comes and goes outside and inside the interpolated tubes so that heat loss is liable to occur, that the catalyst tends to be broken by the movement of the interpolated tubes 14 and the bayonet tubes 13 at the time of startup and shutdown, and that it has an increased shell diameter and a complicated structure so that its scale-up is limited.

### Summary of the Invention

It is an object of the present invention to provide a heat-exchanger type reactor which can absorb the differential thermal expansion between tubes and that between the shell and the tubes in the reactor and is easy to pack and draw out a catalyst.

Other objects of the present invention will be apparent from the following descriptions.

The above-mentioned object of the present invention is achieved by the heat-exchanger type reactor described below.

A heat-exchanger type reactor which comprises tube-side inlet and outlet provided respectively at the upper and lower parts thereof, a plurality of tubes holding a catalyst, upper and lower tube sheets for joining respectively with the upper and lower ends of the tubes, and a reactor shell-section comprising the space partitioned by the outer walls of said tubes, said upper and lower tube sheets and the inner wall of the reactor and having shell-side openings respectively at the upper and lower parts thereof, so that a heat-transfer medium is passed through said reactor shell-section to transfer heat to a reaction fluid flowing through said tubes, wherein the upper ends of said tubes are joined to said upper tube sheet by way of first expansion joints arranged on the upper tube sheet fixed to the inner wall of the reactor, the lower ends of said tubes is directly fixed to the floatable lower tube sheet, a room is formed which is partitioned by said lower tube sheet and an inner end plate (inner head) connected to the lower side thereof and has an opening, and said opening is joined to a tube-side outlet by way of a second expansion joint.

### Brief Description of the Drawings

In the appended drawings:
Fig. 1 is a schematic drawing showing a heat-exchanger type reactor according to the present invention,
Fig. 2 is a schematic drawing showing a structure of the first expansion joint in the present invention, and
Fig. 3 is a schematic drawing showing a conventional reactor employing bayonet tubes.

### Description of the Preferred Embodiments

In the present invention, the lower end of each first expansion joint is fixed to the upper side of the upper tube sheet tied preferably to the upper part of the shell section in the reactor, while the upper end thereof is joined to each tube. The first expansion joints, provided respectively for each of the plural number of tubes, absorb the thermal expansions of the respective tubes, and therefore they can absorb the differential thermal expansion that can occur between tubes, as described above. Further, it is necessary for the first expansion joints to have a strength that can bear the load due to the weight and pressure difference between tube side and shell side.

The upper end of the second expansion joint is joined to an opening provided in the lower part of a room formed between the floatable lower tube sheet and an end plate (head) joined to the lower side thereof, while the lower end thereof is connected to a tube-side outlet of the reactor. The expansion joint absorbs the differential thermal expansion between the reactor shell and the tubes.

The second expansion joint is brought into contact with a reaction fluid coming through the tubes. If the reaction fluid has a high temperature of, for example, 500 °C or above, it is preferable to have a means for preventing the direct contact between the reaction fluid and the joint and/or the radiation heat from the reaction fluid. The means may be, for example, a refractory material so arranged inside the joint as not to restrict the movement of the joint.

The heat-exchanger type reactor according to the present invention can be used to carry out a variety of endothermic or exothermic reactions. As an example of the endothermic reactions may be mentioned the production of a gas rich in hydrogen by steam reforming of hydrocarbons such as natural gas and naphtha (reaction fluid). The heat-transfer medium used herein may include a high-temperature process gas, for example, a process gas having a temperature as high as 830 - 880°C obtained by the steam reforming of hydrocarbons using a combustion gas as a heat source, a process gas having a temperature as high as 900 - 1,100°C obtained by partially oxidizing the foregoing process gas further, or a combustion waste gas having been used as a heat source for the steam reforming, or the combustion gas itself. Among examples using exothermic reactions are ammonia synthesis and methanol synthesis. The heat-transfer medium used herein may be a cooling medium such as a cold feed gas prior to being subjected to the synthesis.

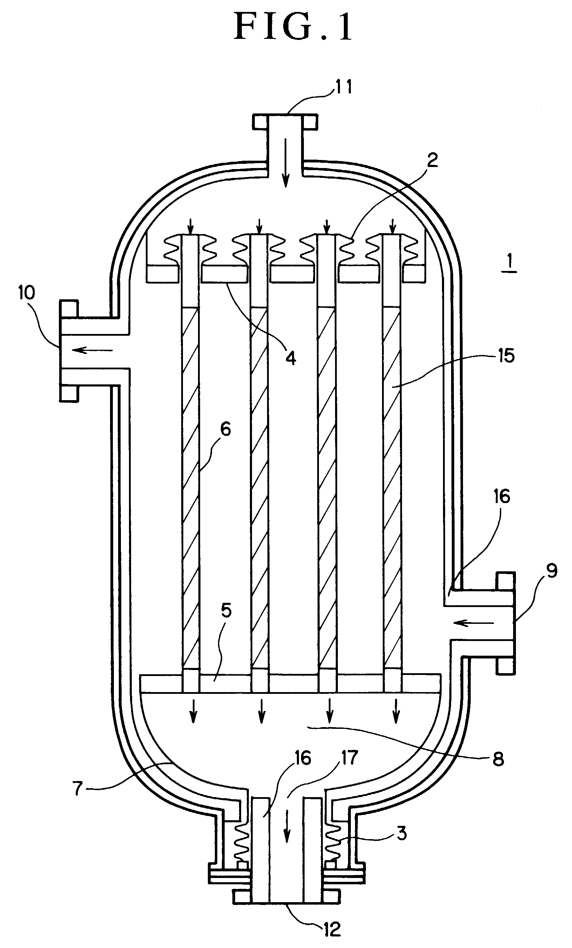

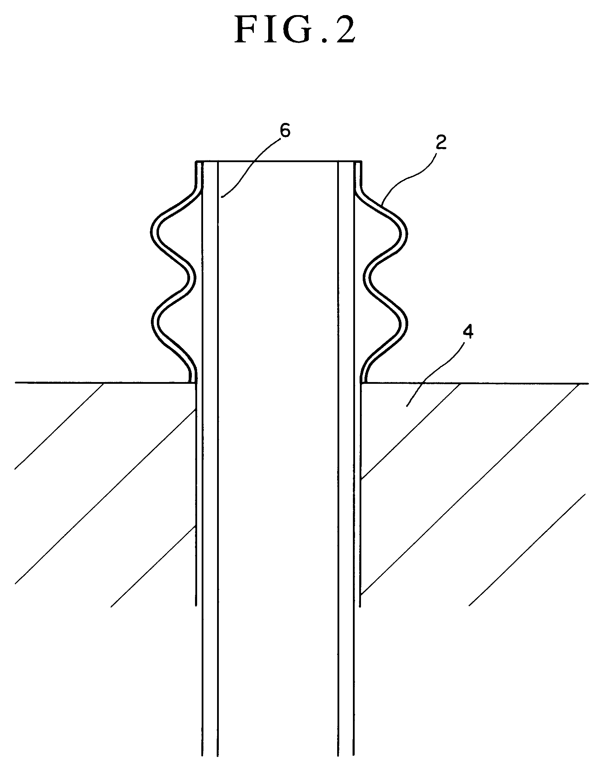

The present invention is specifically illustrated with reference to Fig. 1 and 2 hereunder. Referring to Fig. 1, an upper tube sheet 4 and a lower tube sheet 5 are provided respectively at the upper and lower parts in a generally vertical cylindrical reactor 1.

The upper tube-sheet 4 may be of a flat shape, or of an upward or downward spherical shape to cope with a heavy and large tube bundle. Where the operating temperature is not so high, the upper tube sheet 4 can be fixed directly to the reactor. However, when the operating temperature is so high that the thermal stress at the fitted part is high, the sheet 4 can be fixed to the reactor by using a proper means that can relieve the thermal stress.

The lower tube sheet 5 (having the same shape as that of the upper tube sheet 4) is floatable and forms a floatable room 8 together with an end plate (head) 7 joined to the lower side thereof. An opening 17 is provided in the lower part of the room 8.

The tubes 6 holds a catalyst in the inside thereof. Although the tubes 6 are each shown as a vertically placed straight tube in Fig. 1, they are usually tied up in a required number to form a tube bundle so as to obtain a required reaction amount. The upper end of the tube bundle is joined to the upper tube sheet by way of a first expansion joint 2 for each of the tubes.

The first expansion joints 2 are mounted preferably on the upper side of the upper tube sheet 4 so as not to be exposed directly to high temperatures. However, where an exothermic reaction is carried out and the heat-transfer medium is a cooling medium, the joint 2 may be arranged beneath the lower side of the upper tube sheet 4.

The lower end of the above-described tube bundle is connected directly to the lower tube sheet 5. The second expansion joint 3 is joined at the upper end thereof to the opening 17 in the lower part of the room 8 (the lower part of an inner end plate (head) 7), while the lower end of the joint 3 is connected to the tube-side outlet 12, so that the movement of the room 8 due to thermal expansion may not be restrained. Similarly to the connection between the upper end of the tube bundle and the upper tube sheet 4, it is considered to connect the lower end of the tube bundle to the lower tube sheet 5 by way of expansion joints. In this case, however, the expansion joints are exposed to high temperatures, and it is difficult to design a structure that absorbs the differential thermal expansion between the shell and the tubes. Accordingly, the connections employed in the present invention are superior.

By adopting such a structure, the upper and lower ends of the tube bundle are fitted to the reactor 1 by way of the expansion joints, and hence the tube bundle assumes a floating state against the reactor 1. In addition, both of the first and second expansion joints can be laid in places where the temperature is lowest in the reactor.

It is necessary for the first expansion joints to have a flexibility that can absorb the differential thermal expansion between tubes 6 and a strength that can bear the total load of the tube bundle and the load due to pressure difference. The material of the joint 2 may include high chromium-nickel heat-resistant alloy steels, such as common stainless steels including SUS 304 and 316, Inconel (trade name) and Incolloy (trade name). However, it goes without saying that the material of the joint 2 is not limited to only these materials. The number of crests in the expansion joint 2 is preferably 1 - 4, but is not necessarily limited to these numbers. No particular limitation is imposed on the pitch between crests in the expansion joint. After all, it will suffice if the first expansion joint chooses conditions that can perform the functions demanded to the joint.

The second expansion joint 3 requires a sufficient flexibility to absorb a large differential thermal expansion between the shell of the reactor 1 and the tubes 6. The joint may use as a material a high chromium-nickel heat resistant alloy steel, such as Inconel (trade name) and Incolloy (trade name), in addition to common stainless steels such as SUS 304 and 316, similarly to the material of the first expansion joint. However, needless to say, the material of the joint 3 is not limited only to these materials. The number of crests in the second expansion joint 3 is preferably 5 - 10, but is not necessarily limited to these numbers. No particular limitation is placed on the pitch between crests in the expansion joint. After all, it will suffice if the second expansion joint chooses conditions that can perform the functions demanded to the joint.

In the present invention, there are generated in the tube bundle an downward pushing force due to the pressure drop of a reaction fluid passing through the tubes 6 and an upward or downward pushing force in accordance with whether the total cross-sectional area of the tubes is larger or smaller than the cross-sectional area of the expansion joint 3 by the pressure difference between the inside and outside of the floatable room 8. However, it is possible to balance the forces working on the tube bundle and prevent an unnecessary load from working on the expansion joints 2 by choosing properly the inner diameter of the second expansion joint 3.

Fig. 2 shows an most simple example of the first expansion joint 2, which is molded and fabricated from a single plate. So long as the expansion joint 2 has a structure that can maintain air tightness and absorb the difference in length of the respective tube due to differential thermal expansion, it is not limited to the structure given in Fig. 2. Ambient temperature is relatively low in the place where the first expansion joints are installed, and the amount of expansion to be absorbed may take a value of about 10 mm for the expected number of start-up and shutdown cycle. In view of the weight of the tube bundle and pressure difference to be supported and the expected number of start-up and shutdown cycle, the joint 2 can be designed in fully realistic dimensions.

Regarding the second expansion joint 3, ambient temperature is relatively low in the place where it is installed, and the expansion joint has no dimensional restrictions owing to the installing site in the design of the joint. Therefore, even if the amount of expansion to be absorbed is as large as about 100 mm, the expansion joint can be designed according to ordinary specifications.

Design examples of the first and second expansion joints are illustrated hereinbelow.

### Design example of first expansion joint

### Example 1

| material | | heat-resistant alloy steel |
|---|---|---|
| tube outer diameter | Dt | 114 mm |

| expansion joint | | |
|---|---|---|
| outer diameter | Do | 150 mm |
| inner diameter | Di | 116 mm |
| plate thickness | t | 1 mm |
| pitch | b | 30 mm |
| number of crest | n | 3 |
| supporting load of the joint | | 250 kgf |
| (tube load, catalyst load, differential pressure load) | | |
| allowable amount of expansion of the load | | 10 mm |
| (designed cycle number of shutdown repetition : 300) | | |

### Example 2

| material | | heat-resistant alloy steel |
|---|---|---|
| tube outer diameter | Dt | 141 mm |

| expansion joint | | |
|---|---|---|
| outer diameter | Do | 220 mm |
| inner diameter | Di | 150 mm |
| plate thickness | t | 2 mm |
| pitch | b | 60 mm |
| number of crest | n | 2 |
| supporting load of the joint | | 400 kgf |
| (tube load, catalyst load, differential pressure load) | | |
| allowable amount of expansion of the joint | | 15 mm |
| (designed cycle number of shutdown repetition : 300) | | |

### Design example of second expansion joint

| material | | heat-resistant alloy steel |
|---|---|---|
| expansion joint | | |
| outer diameter | Do | 750 mm |
| inner diameter | Di | 600 mm |
| plate thickness | t | 2 mm |
| pitch | b | 60 mm |
| number of crest | n | 6 |
| allowable amount of expansion of the joint | | 120 mm |
| (designed cycle number of shutdown repetition : 300) | | |

In case of need, it is possible for the second expansion joint 3 to have a structure that can isolate from heat by means of an insulating material 16 as shown in Fig. 1, so that the joint may not be exposed to high temperatures.

A reaction fluid is fed into a reactor 1 through a tube-side inlet 11 at the upper part of the reactor, sent through the inside of tubes 6 with a catalyst packed, and during this time, heat-transferred with a heat-transfer medium, which is introduced through a shell-side opening 9, passed through the shell-section and discharged through a shell-side opening 10, to proceed the reaction by the action of the catalyst. Where the reaction is exothermic, a heat-transfer medium is introduced through the shell-side opening 10 and discharged through the shell-side opening 9. The reaction product stream having completed the reaction is passed through a floatable room 8 and discharged through a tube-side outlet 12 to the outside of the reactor.

Where the heat transfer with the heat-transfer medium flowing through the shell-section is insufficient, it is possible to install fins on the outer sides of the tubes 6 or to cover the tubes 6 with tubes of a little larger diameter and let the heat transfer medium flow through the clearances between said tubes and the tubes 6 to promote the heat transfer.

The present invention has been illustrated as above. As a matter of course, it is possible to apply to the present invention a variety of modifications known to persons skilled in the art, unless the modifications change the gist of the present invention.

The present invention gives the effects as described below.

Firstly, the reactor of the present invention can absorb the differential thermal expansion between the reactor shell and the tubes. Secondly, as the characteristics of a heat-exchanger type reactor, the reaction and heat-transfer conditions vary due to the tolerance in tube inner diameter, the difference in catalyst packing density, the difference in catalyst activity, the uneven distribution of a reaction fluid flowing through the tubes, the uneven distribution of a heat-transfer medium flowing through the shell-section, and the like. Thus, the reaction and heat-transfer conditions differ even in each tube to produce a temperature difference between tubes. The reactor of the present invention can absorb the differential thermal expansion between tubes due to this temperature difference. Thirdly, the function of expansion joints is divided in two, which are placed individually at the upper and lower ends of the tube bundle and in sites where the ambient temperature is lower, so that the reliability of the expansion joints is improved. Fourthly, the expansion joints reduce the tube load generated by the pressure-caused deflections of the upper and lower tube sheets, the deflections being a problem of large-scale reactors. Fifthly, all of the tubes are straight tubes so that packing and withdrawal of a catalyst is easy.

## Claims

1. A heat-exchanger type reactor which comprises tube-side inlet and outlet provided respectively at the upper and lower part thereof, a plurality of tubes holding a catalyst, upper and lower tube sheets for joining respectively with the upper and lower ends of the tubes, and a reactor shell section comprising the space partitioned by the outer walls of said tubes, said upper and lower tube sheets and the inner wall of the reactor and having shell-side openings respectively at the upper and lower parts thereof so that a heat-transfer medium is passed through said reactor shell section to transfer heat to a reaction fluid flowing through said tubes, wherein the upper ends of said tubes are joined to said upper tube sheet by way of first expansion joints arranged on the upper tube sheet fixed to the inner wall of the reactor, the lower ends of said tubes is directly fixed to the floatable lower tube sheet, a room is formed which is partitioned by said lower tube sheet and an inner end plate (inner head) connected to the lower side thereof and has an opening, and said opening is joined to a tube-side outlet by way of a second expansion joint.

2. The reactor according to Claim 1, wherein said first expansion joints are structurally fixed to the upper side of said upper tube sheet at the lower ends and to said tubes at the upper ends to absorb the differential thermal expansion between tubes and bear the total weight and pressure difference between the tube side and shell side.

3. The reactor according to Claim 1, wherein said second expansion joint is structurally fixed to said opening at the upper end and to said tube-side outlet at the lower end to absorb the differential thermal expansion between said shell and said tubes.

4. The reactor according to Claim 1 or 3, which has a means for shielding said second expansion joint from a high-temperature reaction fluid coming through said opening.

5. The reactor according to Claim 1, wherein an endothermic reaction is carried out in the tubes holding said catalyst and said heat transfer medium is a heating medium selected from a high-temperature process gas or a combustion gas.

6. The reactor according to Claim 5, wherein said endothermic reaction is a steam reforming reaction for hydrocarbons.

7. The reactor according to Claim 1, wherein an exothermic reaction is carried out in the tubes holding said catalyst and said heat-transfer medium is a cooling medium.

8. The reactor according to Claim 7, wherein said exothermic reaction is a reaction for synthesizing ammonia or methanol, and said cooling medium is a feed gas prior to being subjected to said reaction.
